# EUROPEAN PATENT SPECIFICATION

(11) **EP 1 513 525 B1**
(45) Date of publication and mention of the grant of the patent: **14.05.2008**
(21) Application number: 03731333.5
(22) Date of filing: 19.06.2003
(51) Int. Cl.: A61K 31/44, A61K 31/4439, A61P 1/04

(54) **USE OF MGLUR5 ANTAGONISTS FOR THE TREATMENT OF GERD**
VERWENDUNG VON MGLUR5 ANTAGONISTEN ZUR BEHANDLUNG VON GASTROESOPHAGALEN REFLUXEN
Utilisation D'ANTAGONISTES Du MGLUR5 POUR LE TRAITEMENT DU REFLUX GASTRO-OESOPHAGIEN

(30) Priority: 20.06.2002 SE 0201943
(43) Date of publication of application: 16.03.2005
(73) Proprietor: AstraZeneca AB, 151 85 Södertälje (SE)
(72) Inventor: LEHMANN, Anders, S-421 66 Västra Frölunda (SE); MATTSSON, Jan, S-429333 Kullavik (SE); STORMANN, Thomas, M. C/o NPS Pharmaceuticals, Inc., Salt Lake City, Utah 84108 (US)
(74) Representative: Plougmann & Vingtoft A/S
(86) International application number: PCT/US2003/016223
(87) International publication number: WO 2004/000316

(56) References cited:
- WO-A-01/12627
- WO-A-99/59612
- US-A- 5 036 057
- US-A- 6 117 908
- GASPARINI F ET AL: "2-METHYL-6-(PHENYLETHYNYL)-PYRIDINE (MPEP), A POTENT, SELECTIVE ANDSYSTEMICALLY ACTIVE MGLU5 RECEPTOR ANTAGONIST" NEUROPHARMACOLOGY, PERGAMON PRESS, OXFORD, GB, vol. 38, no. 10, October 1999 (1999-10), pages 1493-1503, XP001032948 ISSN: 0028-3908
- KNÖPFEL T ET AL: "Metabotropic Glutamate Receptors: Novel targets for drug development" JOURNAL OF MEDICINAL CHEMISTRY, AMERICAN CHEMICAL SOCIETY. WASHINGTON, US, vol. 38, no. 9, 28 April 1995 (1995-04-28), pages 1417-1426, XP000611483 ISSN: 0022-2623

## Description

### Field of the invention

The present invention relates to the use of metabotropic glutamate receptor 5 (mGluR5) antagonists for the inhibition of transient lower esophageal sphincter relaxations. A further aspect of the invention is directed to the use of metabotropic glutamate receptor 5 antagonists for the treatment of gastro-esophageal reflux disease, as well as for the treatment of regurgitation.

### Background of the invention

The metabotropic glutamate receptors (mGluR) are G-protein coupled receptors that are involved in the regulation and activity of many synapses in the central nervous system (CNS). Eight metabotropic glutamate receptor subtypes have been identified and are subdivided into three groups based on sequence similarity. Group I consists of mGluR1 and mGluR75. These receptors activate phospholipase C and increase neuronal excitability. Group II, consisting of mGluR2 and mGluR3 as well as group III, consisting of mGluR4, mGluR6, mGluR7 and mGluR8 are capable of inhibiting adenylyl cyclase activity and reduce synaptic transmission. Several of the receptors also exist in various isoforms, occurring by alternative splicing (Chen, C-Y et al., Journal of Playsiology (2002), 538.3, pp. 773-786*;* Pin, J-P et al., European Journal of Pharmacology (1999), 375, pp. 277-294*;* Bräuner-Osborne, H et al. Journal of Medicinal Chemistry (2000), 43, pp. 2609-2645*;* Schoepp, D.D, Jane D.E. Monn J.A. Neuropharmacology (1999), 38, pp. 1431-1476*).*

The lower esophageal sphincter (LES) is prone to relaxing intermittently. As a consequence, fluid from the stomach can pass into the esophagus since the mechanical barrier is temporarily lost at such times, an event hereinafter referred to as "reflux".

Gastro-esophageal reflux disease (GERD) is the most prevalent upper gastrointestinal tract disease. Current pharmacotherapy aims at reducing gastric acid secretion, or at neutralizing acid in the esophagus. The major mechanism behind reflux has been considered to depend on a hypotonic lower esophageal sphincter. However, e.g. Holloway & Dent (1990) Gastroenterol. Clin. N. Amer. 19, pp. 517-535*,* has shown that most reflux episodes occur during transient lower esophageal sphincter relaxations (TLESRs), i.e. relaxations not triggered by swallows. It has also been shown that gastric acid secretion usually is normal in patients with GERD.

According to Blackshaw L.A. et al., presentation at the conference Neurogastroenterology & Motility, Madison, Wisconsin, 14 November 2001*,* metabotropic glutamate receptors of group II and group III, i.e. mGluR2, mGluR3, mGluR4, mGluR6, mGluR7 and mGluR8 may be involved in selective inhibitory modulation of peripheral mechanosensory endings.

The object of the present invention was to find a new way for the inhibition of transient lower esophageal sphincter relaxations (TLESRs), thereby preventing reflux. More particularly the object of the invention was to find a new and improved way of treating gastro-esophageal reflux disease (GERD), as well as a new and improved way for the treatment of regurgitation.

### Outline of the invention

It has now surprisingly been found that metabotropic glutamate receptor 5 (mGluR5) antagonists are useful for the inhibition of transient lower esophageal sphincter relaxations (TLESRs), and thus for the treatment of gastro-esophageal reflux disease (GERD).

Consequently, the present invention is directed to the use of a metabotropic glutamate receptor 5 antagonist for the manufacture of a medicament for the inhibition of transient lower esophageal sphincter relaxations (TLESRs).

A further aspect of the invention is the use of a metabotropic glutamate receptor 5 antagonist for the manufacture of a medicament for the prevention of reflux. Still a further aspect of the invention is the use of a metabotropic glutamate receptor 5 antagonist for the manufacture of a medicament for the treatment of gastro-esophageal reflux disease (GERD).

Effective prevention of regurgitation would be an important way of preventing, as well as curing lung disease due to aspiration of regurgitated gastric contents, and for managing failure to thrive. Thus, a further aspect of the invention is the use of a metabotropic glutamate receptor 5 antagonist for the manufacture of a medicament for the treatment of regurgitation.

Still a further aspect of the invention is the use of a metabotropic glutamate receptor 5 antagonist for the manufacture of a medicament for the treatment or prevention of reflux-related asthma.

For the purpose of this invention, the term "antagonist" should be understood as including full antagonists, inverse agonists, non-competitive antagonists or competitive antagonists, as well as partial antagonists, whereby a "partial antagonist" should be understood as a compound capable of partially, but not fully, in-activating the metabotropic glutamate receptor 5.

The wording "TLESR", transient lower esophageal sphincter relaxations, is herein defined in accordance with Mittal, R.K., Holloway, R.H., Penagini, R., Blackshaw, L.A., Dent, J., 1995; Transient lower esophageal sphincter relaxation. Gastroenterology 109, pp. 601-610*.*

The wording "reflux" is defined as fluid from the stomach being able to pass into the esophagus, since the mechanical barrier is temporarily lost at such times.

The wording "GERD", gastro-esophageal reflux disease, is defined in accordance with van Heerwarden, M.A., SmoutA.J.P.M., 2000; Diagnosis of reflux disease. Baillière's Clin. Gastroenterol. 14, pp. 759-774*.*

One example of a compound having antagonistic affinity to metabotropic glutamate receptor 5, thereby being useful in accordance with the invention, is the compound 2-methyl-6-(phenylethynyl)-pyridine (often abbreviated MPEP). MPEP is commercially available from e.g. Tocris, or may be synthesized according to well-known procedures such as disclosed by K. Sonogashira et al. in Tetrahedron Lett. (1975), 50, 4467-4470*.*

A further example of a compound having antagonistic affinity to metabotropic glutamate receptor 5, thereby being useful in accordance with the invention, is the compound 3-[3-(5-fluoropyridin-2-yl)-1,2,4-oxadiazol-5-yl]-5-(methoxymethyl)benzonitrile, having the structural formula:

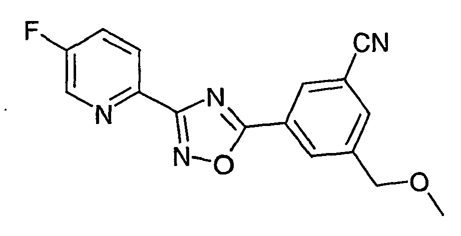

Yet another example of a compound having antagonistic affinity to metabotropic glutamate receptor 5, thereby being useful in accordance with the invention, is the compound 3-fluoro-5-[3-(5-fluoropyridin-2-yl)-1,2,4-oxadiazol-5-yl]benzonitrile, having the structural formula:

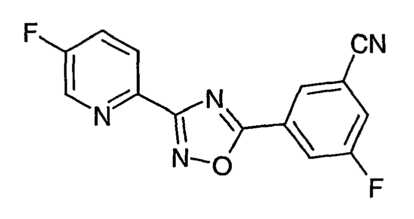

The use of pharmaceutically acceptable salts of metabotropic glutamate receptor 5 antagonists is also within the scope of the present invention. Such salts are for example salts formed with mineral acids such as hydrochloric acid; alkali metal salts such as sodium or potassium salts; or alkaline earth metal salts such as calcium or magnesium salts.

Metabotropic glutamate receptor 5 antagonists having an asymmetric carbon atom are chiral compounds, and depending on the presence of asymmetric atoms, the metabotropic glutamate receptor 5 antagonists may exist in the form of mixtures of isomers, particularly racemates, or in the form of pure isomers such as specific enantiomers. The use of optical isomers of metabotropic glutamate receptor 5 antagonists is also within the scope of the present invention.

### Pharmaceutical formulations

For clinical use, the metabotropic glutamate receptor 5 antagonists are in accordance with the present invention suitably formulated into pharmaceutical formulations for oral administration. Also rectal, parenteral or any other route of administration may be contemplated to the skilled man in the art of formulations. Thus, the metabotropic glutamate receptor 5 antagonists are formulated with at least one pharmaceutically and pharmacologically acceptable carrier or adjuvant. The carrier may be in the form of a solid, semi-solid or liquid diluent.

In the preparation of oral pharmaceutical formulations in accordance with the invention, the metabotropic glutamate receptor 5 antagonist(s) to be formulated is mixed with solid, powdered ingredients such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture is then processed into granules or compressed into tablets.

Soft gelatine capsules may be prepared with capsules containing a mixture of the active compound or compounds of the invention, vegetable oil, fat, or other suitable vehicle for soft gelatine capsules. Hard gelatine capsules may contain the active compound in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance(s) mixed with a neutral fat base; (ii) in the form of a gelatine rectal capsule which contains the active substance in a mixture with a vegetable oil, paraffin oil, or other suitable vehicle for gelatine rectal capsules; (iii) in the form of a ready-made micro enema; or (iv) in the form of a dry micro enema formulation to be reconstituted in a suitable solvent just prior to administration.

Liquid preparations for oral administration may be prepared in the form of syrups or suspensions, e.g. solutions or suspensions, containing the active compound and the remainder of the formulation consisting of sugar or sugar alcohols, and a mixture of ethanol, water, glycerol, propylene glycol and polyethylene glycol. If desired, such liquid preparations may contain colouring agents, flavouring agents, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of a compound of the invention in a pharmaceutically acceptable solvent. These solutions may also contain stabilizing ingredients and/or buffering ingredients and are dispensed into unit doses in the form of ampoules or vials. Solutions for parenteral administration may also be prepared as a dry preparation to be reconstituted with a suitable solvent extemporaneously before use.

In one aspect of the present invention, the metabotropic glutamate receptor 5 antagonists may be administered once or twice daily, depending on the severity of the patient's condition.

A typical daily dose of the metabotropic glutamate receptor 5 antagonists is from 0.1-100 mg per kg body weight of the subject to be treated, but this will depend on various factors such as the route of administration, the age and weight of the patient as well as of severity of the patient's condition.

### Biological evaluation

### Screening for compounds active against TLESR

Adult Labrador retrievers of both genders, trained to stand in a Pavlov sling, are used. Mucosa-to-skin esophagostomies are formed and the dogs are allowed to recover completely before any experiments are done.

### Motility measurement

In brief, after fasting for approximately 17 h with free supply of water, a multilumen sleeve/sidehole assembly (Dentsleeve, Adelaide, South Australia) is introduced through the esophagostomy to measure gastric, lower esophageal sphincter (LES) and esophageal pressures. The assembly is perfused with water using a low-compliance manometric perfusion pump (Dentsleeve, Adelaide, South Australia). An air-perfused tube is passed in the oral direction to measure swallows, and an antimony electrode monitored pH, 3 cm above the LES. All signals are amplified and acquired on a personal computer at 10 Hz.

When a baseline measurement free from fasting gastric/LES phase III motor activity has been obtained, placebo (0.9% NaCl) or test compound is administered intravenously (i.v., 0.5 ml/kg) in a foreleg vein. Ten min after i.v. administration, a nutrient meal (10% peptone, 5% D-glucose, 5% Intralipid, pH 3.0) is infused into the stomach through the central lumen of the assembly at 100 ml/min to a final volume of 30 ml/kg. Immediately following the meal, air is insufflated at 40 ml/min. In an alternative model (Barostat model), the infusion of the nutrient meal is followed by air infusion at a rate of 500 ml/min until a intragastric pressure of 10±1 mmHg is obtained. The pressure is then maintained at this level throughout the experiment using the infusion pump for further air infusion or for venting air from the stomach. The experimental time from start of nutrient infusion to end of air insufflation is 45 min. The procedure has been validated as a reliable means of triggering TLESRs.

TLESRs is defined as a decrease in lower esophageal sphincter pressure (with reference to intragastric pressure) at a rate of >1 mmHg/s. The relaxation should not be preceded by a pharyngeal signal ≤2s before its onset in which case the relaxation is classified as swallow-induced. The pressure difference between the LES and the stomach should be less than 2 mmHg, and the duration of the complete relaxation longer than 1 s.

### EXAMPLES

### Example 1

2-Methyl-6-(phenylethynyl)-pyridine (MPEP) was prepared according to the procedure described by K. Sonogashira et al. in Tetrahedron Lett. (1975), 50, 4467-4470*.* After purification by column chromatography (SiO₂), the hydrochloride salt (the hydrochloride salt of 2-methyl-6-(phenylethynyl)-pyridine is also commercially available from e.g. Tocris) was prepared by introducing HCl(g) to an ice-cooled Et₂O-solution of the product. The hydrochloride salt of MPEP was tested on adult Labrador retrievers of both genders in accordance with the test model described above.

Inhibition of the number of TLESRs was calculated with regard to the five preceding control experiments for each dog, and the results as set out in Tables 1.1 and 1.2 below were achieved.

**Table 1.1 - Standard model**

| **Compound** | **DOSE [µmol/kg]** | **% INHIBITION ± SEM (N)** |
|---|---|---|
| MPEP | 1.4 | 30 ± 5 (4) |
| MPEP | 4.3 | 57 ± 8 (4) |
| MPEP | 8.7 | 59 ± 11 (3) |

| | | |
|---|---|---|
| N = number of dogs tested SEM = standard error of mean | | |

**Table 1.2 - Standard model**

| **Compound** | **DOSE [µmol/kg/h]** *infusion during 60 min* | **% INHIBITION ± SEM (N)** |
|---|---|---|
| MPEP | 4 | 32 ± 13 (4) |
| MPEP | 6 | 43 ± 3 (2) |

| | | |
|---|---|---|
| N = number of dogs tested SEM = standard error of mean | | |

### EXAMPLE 2

3-[3-(5-fluoropyridin-2-yl)-1,2,4-oxadiazol-5-yl]-5-(methoxymethyl)benzonitrile was prepared according to the procedure described in WO 02/068417. 3-[3-(5-fluoropyridin-2-yl)-1,2,4-oxadiazol-5-yl]-5-(methoxymethyl)benzonitrile was tested on adult Labrador retrievers of both genders in accordance with the barostat model described above.

**Table 2.1 - Barostat model**

| **Compound** | **DOSE [µmol/kg/h]** *infusion during* 55 *min* | **% INHIBITION ± SEM (N)** |
|---|---|---|
| 3-[3-(5-fluoropyridin-2-yl)-1,2,4-oxadiazol-5-yl]-5-(methoxymethyl)benzonitrile | 1.1 | 58 ± 11 (4) |
| 3-[3-(5-fluoropyridin-2-yl)-1,2,4-oxadiazol-5-yl]-5-(methoxymethyl)benzonitrile | 2.2 | 83 ± 17 (2) |

| | | |
|---|---|---|
| N = number of dogs tested SEM = standard error of mean | | |

### EXAMPLE 3

3-fluoro-5-[3-(5-fluoropyridin-2-yl)-1,2,4-oxadiazol-5-yl]benzonitrile was prepared according to the procedure described in WO 01/12627. 3-fluoro-5-[3-(5-fluoropyridin-2-yl)-1,2,4-oxadiazol-5-yl]benzonitrile was tested on adult Labrador retrievers of both genders in accordance with the barostat model described above.

**Table 3.1 - Barostat model**

| **Compound** | **DOSE [µmol/kg/h]** *infusion during 60 min* | **% INHIBITION ± SEM (N)** |
|---|---|---|
| 3-fluoro-5-[3-(5-fluoropyridin-2-yl)-1,2,4-oxadiazol-5-yl]benzonitrile | 1 | 70 ± 4 (3) |

| | | |
|---|---|---|
| N = number of dogs tested SEM = standard error of mean | | |

The results shown in the Examples above, support that metabotropic glutamate receptor 5 antagonists are useful for the inhibition of TLESRs, and thus for the treatment of GERD.

## Claims

1. Use of a metabotropic glutamate receptor 5 antagonist, or a pharmaceutically acceptable salt or an optical isomer thereof, for the manufacture of a medicament for the inhibition of transient lower esophageal sphincter relaxations (TLESRs).

2. Use of a metabotropic glutamate receptor 5 antagonist, or a pharmaceutically acceptable salt or an optical isomer thereof, for the manufacture of a medicament for the treatment of gastro-esophageal reflux disease (GERD).

3. Use of a metabotropic glutamate receptor 5 antagonist, or a pharmaceutically acceptable salt or an optical isomer thereof, for the manufacture of a medicament for the prevention of reflux.

4. Use of a metabotropic glutamate receptor 5 antagonist, or a pharmaceutically acceptable salt or an optical isomer thereof, for the manufacture of a medicament for the treatment of, or prevention of, regurgitation.

5. Use of a metabotropic glutamate receptor 5 antagonist, or a pharmaceutically acceptable salt or an optical isomer thereof, for the manufacture of a medicament for the treatment of, or prevention of, reflux-related asthma.

6. Use according to any one of the preceding claims, wherein the metabotropic glutamate receptor 5 antagonist is 2-methyl-6-(phenylethynyl)-pyridine.

7. Use according to claim 6, wherein the metabotropic glutamate receptor 5 antagonist is the hydrochloride salt of 2-methyl-6-(phenylethynyl)-pyridine.

8. Use according to any one of claims 1-5, wherein the metabotropic glutamate receptor antagonist is 3-[3-(5-fluoropyridin-2-yl)-1,2,4-oxadiazol-5-yl]-5-(methoxymethyl)benzonitrile.

9. Use according to any one of claims 1-5, wherein the metabotropic glutamate receptor antagonist is 3-fluoro-5-[3-(5-fluoropyridin-2-yl)-1,2,4-oxadiazol-5-yl]benzonitrile.

10. Use according to any one of the preceding claims, wherein the daily dose of the metabotropic glutamate receptor 5 antagonist is from 0.1-100 mg per kg body weight of the subject to be treated.

## Patentansprüche

1. Verwendung eines Antagonisten des metabotropen Glutamatrezeptors 5 oder eines pharmazeutisch unbedenklichen Salzes oder eines optischen Isomers davon zur Herstellung eines Arzneimittels zur Hemmung der vorübergehenden Entspannungen des unteren Speiseröhren-Schließmuskels (TLESR).

2. Verwendung eines Antagonisten des metabotropen Glutamatrezeptors 5 oder eines pharmazeutisch unbedenklichen Salzes oder eines optischen Isomers davon zur Herstellung eines Arzneimittels zur Behandlung der Gastro-Refluxkrankheit (GERD).

3. Verwendung eines Antagonisten des metabotropen Glutamatrezeptors 5 oder eines pharmazeutisch unbedenklichen Salzes oder eines optischen Isomers davon zur Herstellung eines Arzneimittels zur Prävention von Reflux.

4. Verwendung eines Antagonisten des metabotropen Glutamatrezeptors 5 oder eines pharmazeutisch unbedenklichen Salzes oder eines optischen Isomers davon zur Herstellung eines Arzneimittels zur Behandlung oder Prävention von Regurgitation.

5. Verwendung eines Antagonisten des metabotropen Glutamatrezeptors 5 oder eines pharmazeutisch unbedenklichen Salzes oder eines optischen Isomers davon zur Herstellung eines Arzneimittels zur Behandlung oder Prävention von mit Reflux verbundenem Asthma.

6. Verwendung nach einem der vorhergehenden Ansprüche, wobei der Antagonist des metabotropen Glutamatrezeptors 5 2-Methyl-6-(phenylethinyl)pyridin ist.

7. Verwendung nach Anspruch 6, wobei der Antagonist des metabotropen Glutamatrezeptors 5 das Hydrochloridsalz von 2-Methyl-6-(phenylethinyl)pyridin ist.

8. Verwendung nach einem der Ansprüche 1-5, wobei der Antagonist des metabotropen Glutamatrezeptors 3-[3-(5-Fluorpyridin-2-yl)-1,2,4-oxadiazol-5-yl]-5-(methoxymethyl)benzonitril ist.

9. Verwendung nach einem der Ansprüche 1-5, wobei der Antagonist des metabotropen Glutamatrezeptors 3-Fluor-5-[3-(5-fluorpyridin-2-yl)-1,2,4-oxadiazol-5-yl]benzonitril ist.

10. Verwendung nach einem der vorhergehenden Ansprüche, wobei die Tagesdosis des Antagonisten des metabotropen Glutamatrezeptors 5 von 0,1 bis 100 mg pro kg Körpergewicht des zu behandelnden Patienten beträgt.

## Revendications

1. Utilisation d'un antagoniste du récepteur métabotropique du glutamate de type 5, ou d'un sel ou d'un isomère optique de celui-ci de qualité pharmaceutique, pour la fabrication d'un médicament destiné à l'inhibition des relaxations transitoires du sphincter inférieur de l'oesophage (RTSIO).

2. Utilisation d'un antagoniste du récepteur métabotropique du glutamate de type 5, ou d'un sel ou d'un isomère optique de celui-ci de qualité pharmaceutique, pour la fabrication d'un médicament destiné au traitement du reflux gastro-oesophagien (RGO).

3. Utilisation d'un antagoniste du récepteur métabotropique du glutamate de type 5, ou d'un sel ou d'un isomère optique de celui-ci de qualité pharmaceutique, pour la fabrication d'un médicament destiné à la prévention du reflux.

4. Utilisation d'un antagoniste du récepteur métabotropique du glutamate de type 5, ou d'un sel ou d'un isomère optique de celui-ci de qualité pharmaceutique, pour la fabrication d'un médicament destiné au traitement, ou à la prévention, de la régurgitation.

5. Utilisation d'un antagoniste du récepteur métabotropique du glutamate de type 5, ou d'un sel ou d'un isomère optique de celui-ci de qualité pharmaceutique, pour la fabrication d'un médicament destiné au traitement, ou à la prévention, de l'asthme lié au reflux.

6. Utilisation selon l'une quelconque des revendications précédentes, dans laquelle l'antagoniste du récepteur métabotropique du glutamate de type 5 est la 2-méthyl-6-(phényléthynyl)-pyridine.

7. Utilisation selon la revendication 6, dans laquelle l'antagoniste du récepteur métabotropique du glutamate de type 5 est le sel de chlorhydrate de la 2-méthyl-6-(phényléthynyl)-pyridine.

8. Utilisation selon l'une quelconque des revendications 1 à 5, dans laquelle l'antagoniste du récepteur métabotropique du glutamate est le 3-[3-(5-fluoropyridin-2-yl)-1,2,4-oxadiazol-5-yl]-5-(méthoxyméthyl)benzonitrile.

9. Utilisation selon l'une quelconque des revendications 1 à 5, dans laquelle l'antagoniste du récepteur métabotropique du glutamate est le 3-fluoro-5-[3-(5-fluoropyridin-2-yl)-1,2,4-oxadiazol-5-yl]benzonitrile.

10. Utilisation selon l'une quelconque des revendications précédentes, dans laquelle la posologie quotidienne de l'antagoniste du récepteur métabotropique du glutamate de type 5 varie de 0,1 à 100 mg par kilo de poids corporel du patient à traiter.
